(19) Europäisches Patentamt
European Patent Office
Office européen des brevets

(11) EP 1 537 864 A1

(12) **EUROPÄISCHE PATENTANMELDUNG**

(43) Veröffentlichungstag:
**08.06.2005 Patentblatt 2005/23**

(21) Anmeldenummer: **03027678.6**

(22) Anmeldetag: **03.12.2003**

(51) Int Cl.⁷: **A61K 31/198**, A61K 38/06, A61K 31/10, A61K 31/185, A61K 31/19, A61P 3/10

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(71) Anmelder: **Liebel, Franz-Peter, Dr.
72663 Grossbettlingen (DE)**

(72) Erfinder: **Liebel, Franz-Peter, Dr.
72663 Grossbettlingen (DE)**

(54) **Verwendung von Aminosäuren und schwefeltragenden Verbindungen zur Prophylaxe und Therapie des UDP-Glucuronosyltransferase1-defizitären Diabetes mellitus Typ II**

(57) Eine Teilmenge des Diabetes mellitus, nämlich der UDP-Glucuronosyltransferase1-defizitäre Diabetes mellitus TypII (UGT-Diabetes) wird aufgefaßt als das Resultat unzureichender Entgiftung, die wiederum die Folge leistungsgeminderter Isoenzyme der UDP-Glucuronosyltransferase 1 ist.
Es findet also eine verminderte Glukuronidierung statt, so daß zum Ausgleich vermehrt andere Konjugationsmechanismen herangezogen werden, darunter Sulfatierung, Methylierung, Glutathionkonjugation und Taurinkonjugation. Eine Minderung des Sulfat- und Glutathionpools ist die Folge.
Konsequenterweise werden zur Prophylaxe und Therapie des UGT-Diabetes neben anderen Stoffen zuvorderst Methionin und Acetylcystein daneben auch Lysin und Arginin eingesetzt.

EP 1 537 864 A1

## Beschreibung

A) Verwendbarkeit der Erfindung

Die Erfindung dient zur Prophylaxe und Therapie einer Unterform des Diabetes mellitus, nämlich des UDP Glucuronosyltransferase 1 defizitären Diabetes mellitus Typ II (UGT-Diabetes).
Da bei 8 % der Kaukasier UGT1A1 und damit verknüpft fast immer auch UGT1A6 sowie UGT1A7 jeweils ohne Wildtyp-Allel vorliegen, ist bei diesem Teil der Bevölkerung mit einem hohen Entgiftungsdefizit und einem nicht unerheblichen Anteil an UGT-Diabetes-Fällen zu rechnen.
Die verwendete Stoffmenge erhöht wirksam die Möglichkeiten zur Sulfatierung, Methylierung, Glutathionkonjugation, Taurinkonjugation und Metallothionein-Synthese, und man erreicht so die Entgiftung von Toxinen (Auflistung siehe: Kapitel B), die über direkt Zellschädigung, über Proteinkopplung oder über Ah-Rczcptoren den UGT-Diabetes in Gang setzen.

B) Stand der Technik und Vorteile des Verfahrens

Die Genese des Diabetes mellitus Typ II ist derzeit allenfalls in Teilschritten geklärt Hier wird postuliert, daß eine - mit Sicherheit unvollständig aufgelistete - Auswahl von Toxinen infolge eines bestehenden Defektes der physiologischen Entgiftungsmechanismen in der Lage ist, durch die Schädigung der Strukturen oder der metabolischen Abläufe von Zielzellen, durch die Anheftung an Proteine oder durch die Einflußnahme über Arylhydrocarbon-Rezeptoren (Ah-Rezeptoren) an. der Entstehung des UGT-Diabetes entscheidend mitzuwirken.
Als Toxine kommen unter anderem die in der nachfolgenden Tabelle enthaltenen Substanzen in Betracht. Für die Positionen 1) bis 9) sind Schwefelverbindungen, für 10) bis 13) ist Glukuronsäure das hauptsächliche Mittel zur Aufhebung der Giftwirkung.

| Toxin | Zufuhr durch: | Hauptsächliche Entgiftung: |
|---|---|---|
| 1) Acrylamid | Brot, Gebäck, Kaffee, Kakao, Bratkartoffel | Glutathionkopplung |
| 2) Polychlorierte Biphenyle (PCB) | Milch, Fleisch, Fisch | Glutathionkopplung |
| 3) Polycyclische aromatische Kohlenwasserstoffe (PAK) | Pyrolyseprodukte, Kaffee, Milch, Blattgemüse | Glutathionkopplung, Glukuronidierung, Sulfatierung |
| 4) Arsen | Inhalative Aufnahme | Glutathion antagonisiert |
| 5) Quecksilber | Frischwasser- und Seefische, Meeresfrüchte | Glutathion bindet Hg-Verbindungen |
| 6) Blei | Obst, Gemüse, Atemluf | Cystein |
| 7) Patulin | Mehlprodukte, Fleisch, braunfaule Äpfel | Hohe Affinität zu SH-Gruppen |
| 8) Penicillinsäure | Lebensmittel, Tierfutter | Führt zu Glutathion-Depletion |
| 9) Trichothecene | Getreide, vor allem Weizen, Tierfutter | Hohe Affinität zu SH-Gruppen |
| 10) Dibenzodioxine, Dibenzofurane | Milch, Fleisch, Eier | Nach Hydroxylierung entgiftet durch Glukuronidierung |
| 11) Pentachlorphenol (PCP) | Zufuhr durch verunreinigte Lebensmittel | Glukuronidierung |
| 12) Chlorogensäure, Kaffeesäure, Protocatechusäure, | Möhren, Sellerie, Kaffee, Schwarztee, Kakao, Wein, Kartoffiel, Kopfsalat, Endivie, Kohl, Äpfel, Beeren | Nach der Metabolisierung zu einfachen Phenolen erfolgt Glukuronidierung |

(fortgesetzt)

| Toxin | Zufuhr durch: | Hauptsächliche Entgiftung: |
|---|---|---|
| 13) Cumarine | Getreide, Hülsenfrüchte, Nachtschatten, Fenchel, Citrus, Aprikosen, Erdbeeren, Kirschen, Möhren | Nach der Hydroxylierung erfolgen Glukuronidierung und andere Konjugationen |

Von Vorteil ist es, wenn man für die Toxine, die bevorzugt glukuronidiert werden, durch Sulfatierung, Methylierung, Glutathionkopplung oder Taurinkonjugation doch noch die Eliminierung erreicht. Dies wird ermöglicht durch die erhöhte Bereitstellung von Sulfat, Methylgruppen, Glutathion und Taurin.

Die außerdem noch existierenden Konjugationsmechanismen, nämlich Acetylierung und Konjugation mit Aminosäuren, kommen für eine mögliche Beeinflussung nicht in Betracht, da Substratdefizite wie bei den drei schwefelverbrauchenden Konjugationen kaum zu erwarten sind.

C) Darstellung der Lösung und Beispiel

1. L-Methionin wird unter Mitwirkung von $B_6$, $B_{12}$ und Folsäure zu L-Cystein wie folgt metabolisiert:

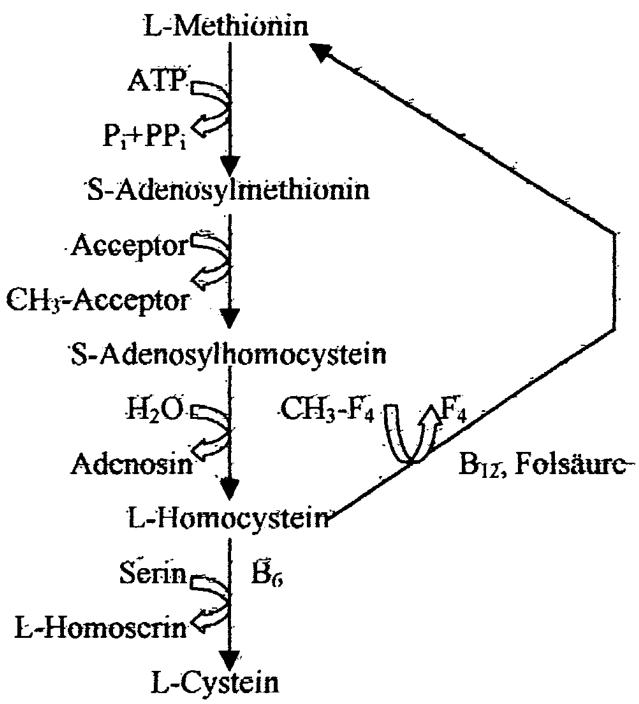


Abb.1: Verstoffwechslung des Methionins zu Cystein.

2. (Acceptor/ $CH_3$-Acceptor) in Ziffer 1. sind hauptsächlich (Guanidinoacetat/ Kreatin).

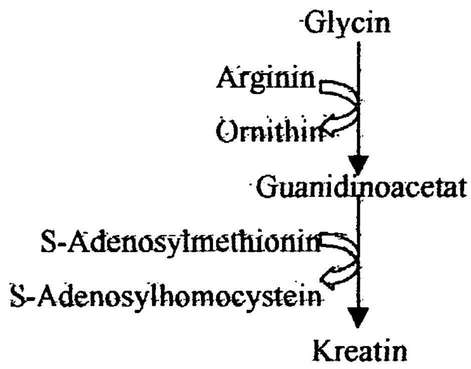


Abb.2: Verbrauch des Adenosylmethionins bei der Synthese von Kreatin.

3.

Für die Hemmung der Harnstoffsynthese zugunsten der in Ziffer 2, gezeigten Kreatinsynthese dient Lysin.

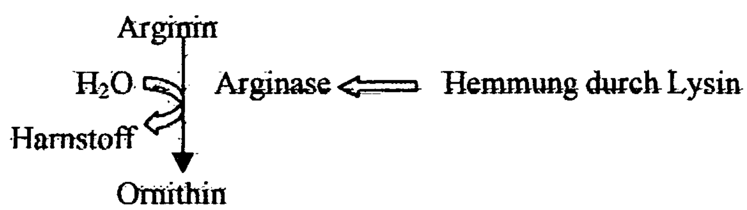


Abb.3: Ausschnitt aus dem Harnstoffcyclus.

4.

Das aus dem Methioninstoffwechsel gewonnene oder durch N-Acetylcystein zugeführte L-Cystein wird wie folgt zu Sulfat und Glutathion umgesetzt:

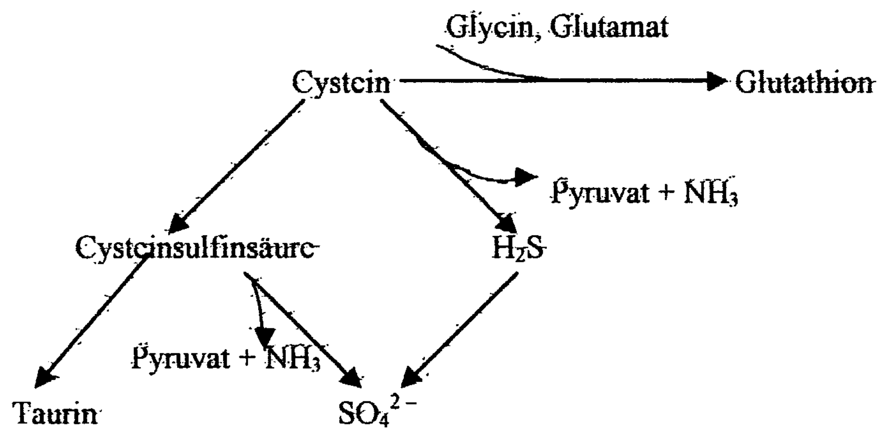


Abb.4: Erzeugung von Sulfat, Glutathion und Taurin aus Cystein.

5.

Am Beispiel der Phenolcarbonsäuren und ihrer Ester werden die Auswirkungen einer mangelhaften Glukuronidierung gezeigt.

Als Phenolcarbonsäuren bezeichnet man Hydroxyzimtsäuren (z.B. o-Cumarsäure, p-Cumarsäure, Kaffeesäure) und Hydroxybenzoesäuren (z.B. Salicylsäure, p-Hydroxybenzoesäure, Protocatechusäure). Ein Ester der Kaffeesäure ist die in vielen Pflanzen enthaltene Chlorogensäure.

Aus den Hydroxyznntsäuren können durch Abbau der Seitenkette über den Weg der β-Oxidation Hydroxybenzoesäuren entstehen, die wiederum durch Decarboxylierung einfache Phenole ergeben.

Für die metabolische Aktivierung des Phenols gilt:

Phenol → Hydrochinon → p-Benzochinon.

Das p-Benzochinon ist dann Ausgangssubstanz des folgenden Schädigungszyklus:

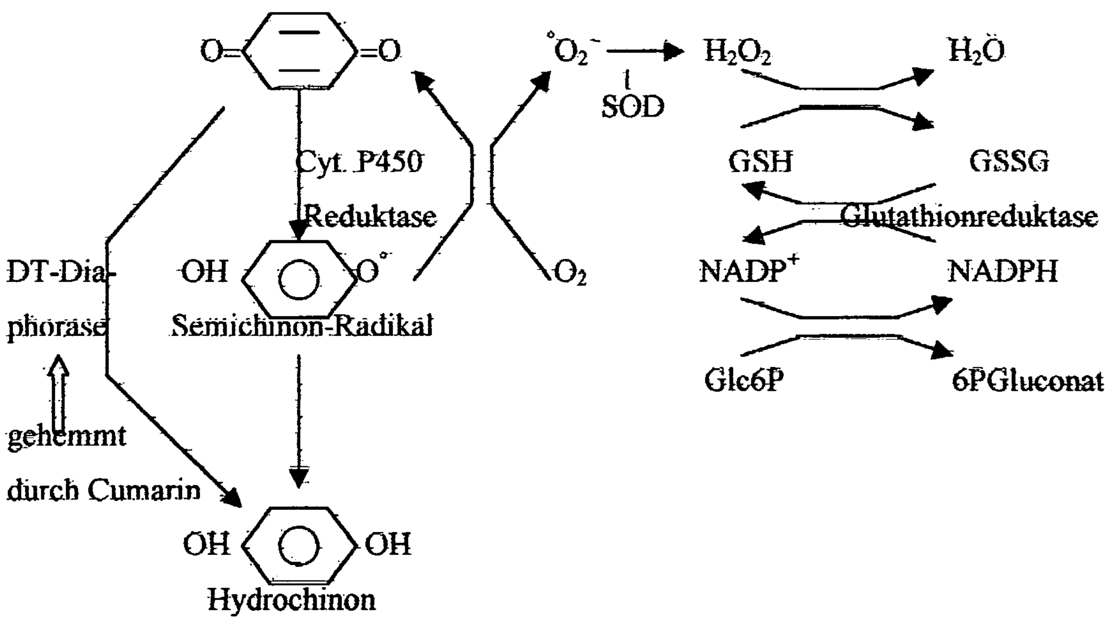


Abb.5: Entgiftung der durch Chinone erzeugten Radikale mittels Glutathion.

Bei mangelnder Phenol-Glukuronidierung infolge eines UGT 1-Defekts sind gem. Abb.5. drei Schädigungsmechanismen festzuhalten:

1.

Die durch den UGT1-Defekt bewirkte unzureichende Glukuronidierung des Phenols muß ersetzt werden durch andere Entgiftungsmechanismen, z.B. durch eine Sulfatierung oder Gtutathionkopplung, bei entsprechendem Verbrauch.

2.

Das nicht oder nur verzögert eliminierte Phenol erzeugt Sauerstoffradikale, aus denen mit Hilfe der Superoxiddismutase $H_2O_2$ entsteht, dessen Metailisierung zu $H_2O$ Glutathion beansprucht und so den Glutathionpool weiter mindert.

3.

Cumarine, die infolge der generell verminderten Glukuronidierung erhöht vorliegen, und die durch Hemmung der DT-Diaphorase die unmittelbare Bildung von Hydrochinon aus p-Benzochinon behindern, erhöhen die Belastung mit Semichinon-Radikalen; die Eliminierung der Radikale steigert den Glutathionverbrauch weiter.

Die Bedeutung und die Wirkung der im Patentanspruch aufgelisteten Substanzen wird wie folgt zusammengefaßt:

<u>Methionin:</u>

Die Aminosäure Methionin ist Ausgangssubstanz eines zu Cystein führenden Stoffwechslwegs, wobei gleichzeitig über S-Adenosylmethionin die Methylgruppen für Methylierungen bereitgestellt werden (siehe Abb.1); aus dem gewonnenen Cystein entstehen Glutathion, Taurin und Sulfat (siehe Abb. 4).

Lysin und Arginin:

Lysin hemmt die Arginase des Harnstoffzyklus, so daß - zusammen mit dem exogenen Arginin - vermehrt Arginin für die Kreatinsynthese zur Verfügung steht. Damit hat der Methioninstoffwechsel in ausreichender Menge einen $CH_3$-Acceptor, nämlich Guanidinoacetat, wodurch Methionin ohne Hemmnisse abfließen kann. (Siehe Abb. 1, 2 und 3.)

N-Acetylcystein:

N-Acetylcystein erhöht nach Deacetylierung den Pool an Cystein, das die Versorgung mit Glutathion, Sulfat und Taurin sicherstellt. (Siehe Abb 4.)

Glutathion und Taurin:

Die Zufuhr von Glutathion, das als Tripeptid u.U. ohne Proteolyse die Darmwand passieren kann, sowie von Taurin ermöglicht es, daß Cystein eingespart und der leicht zu erschöpfende Sulfatpool begünstigt werden kann. Eventuell erreichte hohe Taurin- und Glutathionspiegel bringen einen weiteren Vorteil, da Taurin und Glutathion - neben ihrer Befähigung zur Konjugatbildung - auch direkt mit Radikalen wie $^{\circ}O_2^-$ reagieren.

Cystin

Außerhalb der Zelle existiert Cystein nur in oxidierter Form als Disulfid Cystin, aus dem durch hydrierende Spaltung der Disulfidbrücke mittels Cystinreduktase Cystein gewonnen werden kann. Die intestinale Resorption von Cystin konkurriert mit der Resorption von Lysin und Arginin, da die drei Aminosäuren infolge ihrer strukturellen Ahnlichkeit ein gemeinsames Transportsystem benutzen.

Selbstverständlich begünstigen eine Reihe von Vitaminen die Bereitstellung der schwefeltragenden Verbindungen und die Eliminierung von Radikalen. Für die nachfolgend aufgelisteten Vitamine, deren ausreichende Zufuhr von anderen Stellen zur Besserung unterschiedlichster metabolischer Störungen empfohlen werden (auch zur Besserung von Diabetes in Form sogenannter "Diabetiker-Vitamine"), ergeht kein Patentanspruch sondern lediglich ein informativer Hinweis.

$B_6$, $B_{12}$ und Folsäure:

Der Methioninstoffwechsel benötigt $B_6$ für die Erzeugung von Cystein sowie $B_{12}$ und Folsäure für die Remethylierung. (Siehe Abb. 1.)

Vitamine E und C:

Radikale und chinoide Verbindungen werden durch $\alpha$-Tocopherole (Vitamin E) und Ascorbinsäure (Vitamin C) reduziert. Die oxidierte Ascorbinsäure steht über Glutathion und die Ascorbatreduktase in Redoxbeziehung zu NADPH.

Vitamin $B_1$:

Ein Mangel an Vitamin $B_1$ gefährdet die Funktion der Transketolase, dadurch den Durchsatz durch den Hexosemonophosphatweg und so die ausreichende Erzeugung von NADPH, das Substrat der Glutathionreduktase ist. (Siehe Abb. 5.)

Riboflavin:

Riboflavinmangel ist charakterisiert durch eine Beeinträchtigung der Funktion des Flavoproteins Glutathionreduktase. (Siehe Abb. 5.)

Bemerkungen:

* Aus Verlaufsbeobachtungen hat sich eine günstige Einflußnahme von Vitamin $B_1$ herausgestellt, die noch nicht eindeutig zugeordnet werden kann.
* Methionin kann nur teilweise durch Cystein ausgeglichen werden, da dieses die proteinogene und methylierende Funktion des Methionins nicht ersetzt Zudem soll die Gabe von Methionin stets zusammen mit Lysin und Arginin vorgenommen werden, da sich Methionin bei isolierter Zufuhr als giftig erweist
* Neben einer verminderten Einnahme toxinhaltiger Nahrung ist für UGT Diabetiker intensives Muskeltraining zu empfehlen, da das in der Ablaufskizze des Methioninstoffwechsels erscheinende Paar (Acceptor / $CH_3$-Acceptor) neben anderen Paarungen hauptsächlich (Guanidinoacetat / Kreatin) meint, und da die Ausscheidung überschüssigen Kreatinins direkt proportional zur Muskelmasse ist.

Es bleibt zu klären, welcher pathogenetischer Mechanismus dazu führt, daß eine verminderte Glukuronidierung einen UGT-Diabetes und weitere häufig damit verknüpfte Krankheitsbilder erzeugen kann. Solche begleitenden, aber auch isoliert auftretenden Erkrankungen, sie sind im Patentanspruch im einzelnen genannt, treten deshalb häufig zusammen mit dem UGT-Diabetes auf, da sie ganz ohne Zweifel gemeinsame Ursachen besitzen. Aus den vorangegangenen Aussagen und Zusammenhängen ergibt sich als Hypothese die nachfolgende kompakt gefaßte Ablaufskizze für die Pathogenese des UGT-Diabetes und seiner Begleiterkrankungen.

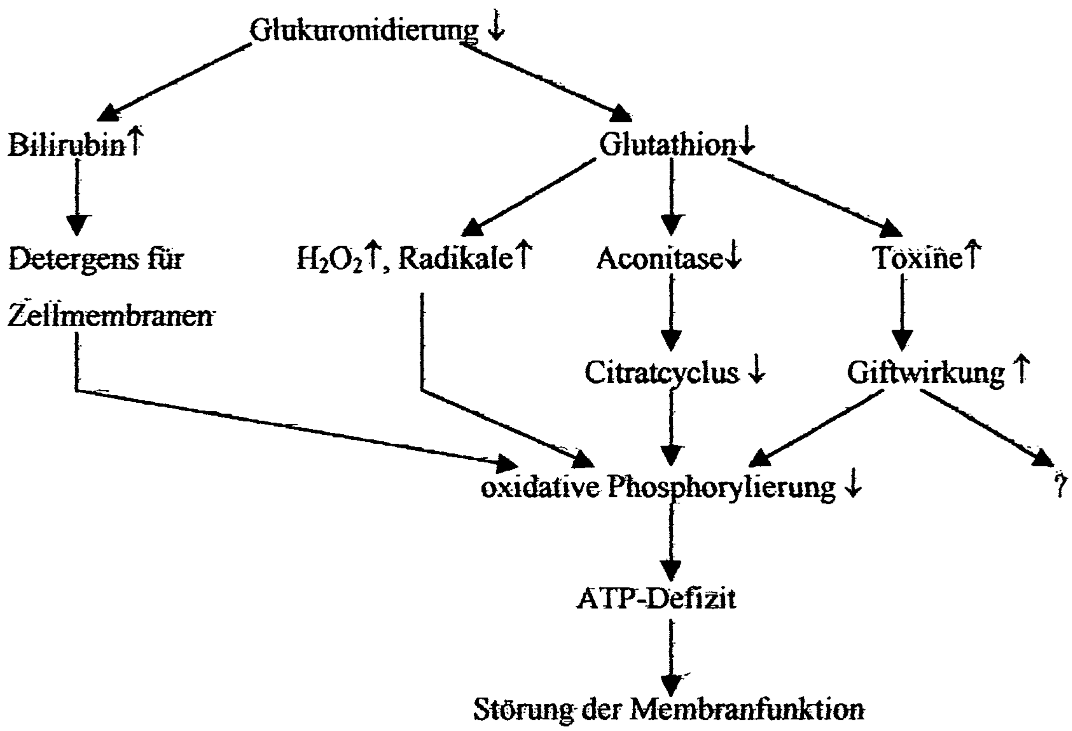


Abb.6: Pathogenese des UGT-Diabetes und assoziierter Erkrankungen.

Unter Beachtung der Tatsache, daß Bilirubin ein sehr wirksamer Radikalenfanger ist, wirkt sich ein aktivitätsgemindertes UGT1A1, das Bilirubin nicht ausreichend glukuronidiert, mildernd aus im Hinblick auf die Auswirkungen eventuell leistungsschwacher UGT1-Isoenzyme UGT1A6 und UGT1A7, die phenotische Verbindungen unzureichend gtokuronidieren und u.a. Radikale generieren.

Diese Begünstigung wird wieder aufgewogen durch die Bindung des Bilirubins an das in Membranen enthaltene Lecithin und seine (in Abb 6 eingetragene) Wirkung als Detergens.

## Patentansprüche

1. Die Erfindung ist dadurch charakterisiert, daß eine Teilmenge des Diabetes mellitus definiert wird als das Resultat unzureichender Glukuronidierung durch Isoenzyme der UDP-Glucuronosyltransferase 1, und daß zur Prophylaxe und Therapie dieses sogenannten UDP-Glucuronosyltransferasel-defizitären Diabetes mellitus Typ II (UGT-Diabetes) möglichst frühzeitig, insbesondere wenn

   - Bilirubin und/oder
   - Glutathionreduktase zu hoch sowie
   - Glutathion zu niedrig

   getestet werden, spätestens aber bei diagnostiziertem Diabetes mellitus Typ II, eine molekulargenetische Überprüfung der UDP-Glucuronosyltransferase 1 (UGT1) erfolgt, wobei nach derzeitigen Erkenntnissen 9 Isoenzyme zur Überprüfung anstehen und wobei insbesondere das Vorliegen der UGT 1-Polymorphismen

   - UGT1A1*28,
   - UGT1A6*2,
   - UGT1A6 R184S,
   - UGT1A7*2 und
   - UGT1A7*3

   getestet werden soll, und daß dann bei festgestellter Aktivitätsminderung von UGT1-Isoenzymen zur Prophylaxe und Therapie des langfristig sich daraus entwickelnden UGT-Diabetes sowie zur Prophylaxe und Therapie der häufig damit assoziierten, jedoch auch isoliert auftretenden Erkrankungen, insbesondere

   - der peripheren Neuropathie,
   - der distalen Muskelatrophie,
   - der männlichen Sexualfunktionsstörungen,
   - der extremen Muskelermüdbarkeit und
   - der Lipidstoffwechselstörungen,

   gleichzeitig in ungefähr äquimolaren Mengen die drei Aminosäuren

   - L-Methromin,
   - L-Lysin und
   - L-Arginin

   sowie.

   - N-Acetylcystein

   zugeführt werden, ergänzt durch eine dem Bedarf angepaßte Auswahl aus

   - Glutathion,
   - L-Cystin und
   - Taurin,

   wobei die Zufuhr von Methionin durch Weizen, von Lysin und Arginin durch Bohnen bzw. Erbsen spezifisch erhöht werden kann, und wobei als eine in weiten Grenzen variable Empfehlung dienen mag, morgens und abends 200 mg Acetylcystein, mittags jeweils 500 mg der Aminosäuren Methionin, Lysin und Arginin sowie ergänzend eine Auswahl der übrigen Stoffe in jeweils 100 mg-Mengen einzunehmen, und diese Startversion unter Beachtung der Methioningegenanzeigen sowie der Leber- und Nierenfunktion so zu variieren, daß Glutathion und der mit dem Urin ausgeschiedene anorganische Schwefel deutlich im oberen Normbereich, die Glutathionreduktase innerhalb der Normgrenzen getestet werden.

**Europäisches Patentamt**

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung
EP 03 02 7678

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.7) |
|---|---|---|---|
| Y | US 2003/078269 A1 (PEARSON DON C ET AL) 24. April 2003 (2003-04-24)<br>* Ansprüche 1,4 *<br>--- | 1 | A61K31/198<br>A61K38/06<br>A61K31/10<br>A61K31/185<br>A61K31/19<br>A61P3/10 |
| Y | WO 02/102360 A (DIOGUARDI FRANCESCO SAVERIO ;CONTI FRANCO (IT); PROFESSIONAL DIETE) 27. Dezember 2002 (2002-12-27)<br>* Ansprüche 1,3,5 *<br>--- | 1 | |
| A | WO 02/13814 A (REMACLE CLAUDE ;UNIV LOUVAIN (BE); REUSENS BRIGITTE (BE); HILL DAV) 21. Februar 2002 (2002-02-21)<br>* Ansprüche 10-12 *<br>--- | 1 | |
| A | WO 98/35667 A (TURNER NICHOLAS CHARLES ;PIERCY VALERIE (GB); SMITHKLINE BEECHAM P) 20. August 1998 (1998-08-20)<br>* Ansprüche 1,2,4 *<br>---<br>-/-- | 1 | |

RECHERCHIERTE SACHGEBIETE (Int.Cl.7)

A61K

## UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

| Recherchenort | Abschlußdatum der Recherche | Prüfer |
|---|---|---|
| DEN HAAG | 14. Mai 2004 | Koch, J |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C09)

Europäisches Patentamt

**EUROPÄISCHER TEILRECHERCHENBERICHT**

Nummer der Anmeldung
EP 03 02 7678

| EINSCHLÄGIGE DOKUMENTE | | | KLASSIFIKATION DER ANMELDUNG (Int.Cl.7) |
|---|---|---|---|
| Kategorie | Kennzeichnung des Dokuments mit Angabe, soweit erforderlich der maßgeblichen Teile | Betrifft Anspruch | |
| A | JEFFREY C. STEVENS ET AL.: "Characterization of 2-[[4-[[2-(1H-Tetrazol-5-ylmethyl)phenyl]methoxy]phenoxy]methyl] Quinoline N-Glucuronidation by in vitro and in vivo approaches" DRUG METABOLISM AND DISPOSITION, Bd. 29, Nr. 3, 2001, Seiten 289-295, XP002276743 USA * Zusammenfassung * --- | 1 | |
| A | US 2003/077339 A1 (YOON JI-WON ET AL) 24. April 2003 (2003-04-24) * Ansprüche 1,18,19 * --- | 1 | RECHERCHIERTE SACHGEBIETE (Int.Cl.7) |
| A | EP 0 956 867 A (LIEBEL FRANZ PETER DR) 17. November 1999 (1999-11-17) * Ansprüche 1-7 * ----- | 1 | |

5

EPO FORM 1503 03.82 (P04C12)

Europäisches
Patentamt

UNVOLLSTÄNDIGE RECHERCHE
ERGÄNZUNGSBLATT C

Nummer der Anmeldung
EP 03 02 7678

Obwohl Anspruch 1 sich auf ein Verfahren zur Behandlung des menschlichen/tierischen Körpers bezieht (Artikel 52(4) EPÜ), wurde die Recherche durchgeführt und gründete sich auf die angeführten Wirkungen der Verbindung/Zusammensetzung.

--------------------

Unvollständig recherchierte Ansprüche:
1

Grund für die Beschränkung der Recherche (nicht patentfähige Erfindung(en)):

Artikel 52 (4) EPÜ - Verfahren zur therapeutischen Behandlung des menschlichen oder tierischen Körpers

**ANHANG ZUM EUROPÄISCHEN RECHERCHENBERICHT ÜBER DIE EUROPÄISCHE PATENTANMELDUNG NR.** EP 03 02 7678

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-05-2004

| Im Recherchenbericht angeführtes Patentdokument | Datum der Veröffentlichung | Mitglied(er) der Patentfamilie | Datum der Veröffentlichung |
|---|---|---|---|
| US 2003078269 A1 | 24-04-2003 | KEINE | |
| WO 02102360 A | 27-12-2002 | IT TO20010580 A1 | 16-12-2002 |
| | | CA 2448745 A1 | 27-12-2002 |
| | | EP 1399139 A2 | 24-03-2004 |
| | | WO 02102360 A2 | 27-12-2002 |
| WO 0213814 A | 21-02-2002 | WO 0213813 A1 | 21-02-2002 |
| | | AU 6551600 A | 25-02-2002 |
| | | AU 8373901 A | 25-02-2002 |
| | | WO 0213814 A1 | 21-02-2002 |
| | | CA 2386985 A1 | 21-02-2002 |
| | | CA 2416440 A1 | 21-02-2002 |
| | | EP 1309321 A1 | 14-05-2003 |
| | | US 2003180345 A1 | 25-09-2003 |
| WO 9835667 A | 20-08-1998 | WO 9835667 A1 | 20-08-1998 |
| | | AU 1725197 A | 08-09-1998 |
| | | CA 2280871 A1 | 20-08-1998 |
| | | EP 0957909 A1 | 24-11-1999 |
| | | JP 2001512450 T | 21-08-2001 |
| US 2003077339 A1 | 24-04-2003 | US 6551627 B1 | 22-04-2003 |
| | | BR 0205144 A | 06-05-2003 |
| | | CN 1462189 T | 17-12-2003 |
| | | EP 1399171 A1 | 24-03-2004 |
| | | WO 02089825 A1 | 14-11-2002 |
| EP 0956867 A | 17-11-1999 | EP 0956867 A1 | 17-11-1999 |

EPO FORM P0461

Für nähere Einzelheiten zu diesem Anhang : siehe Amtsblatt des Europäischen Patentamts, Nr.12/82